# EUROPÄISCHE PATENTANMELDUNG

(11) **EP 0 645 082 A1**
(43) Veröffentlichungstag der Anmeldung: **29.03.1995**
(21) Anmeldenummer: 94113274.8
(22) Anmeldetag: 25.08.1994
(51) Int. Cl.: A01N 35/02, C02F 1/50

(54) **Mittel zum Dotieren wässriger Systeme mit Acrolein**

(30) Priorität: 24.09.1993 DE 4332586
(71) Anmelder: Degussa Aktiengesellschaft, D-60311 Frankfurt (DE)
(72) Erfinder: Werle, Peter, Dr., D-63571 Gelnhausen (DE); Pahling, Werner, D-61184 Karben (DE); Trageser, Martin, D-63571 Gelnhausen (DE)

(57) **Zusammenfassung**

Zum Dotieren wäßriger Systeme mit Acrolein in biozid wirksamer Konzentration wird flüssiges Acrolein verwendet, dessen Gefahreneigenschaften aber die Handhabung erschweren.

Die leichter und sicherer handhabbaren erfindungsgemäßen Mittel sind gekennzeichnet durch einen Gehalt an 30 bis 95 Gew.-% Acrolein und 5 bis 70 Gew.-% anorganischen Trägermaterialien; bevorzugte Trägermaterialien sind Kieselsäuren. Vorzugsweise liegt das pulverförmige oder hochviskose Stoffgemisch in geformter Form vor und ist zusätzlich mit einer acroleindampfdichten Umhüllung versehen.

Zum Dotieren wäßriger Systeme werden die Mittel direkt in das Wasser eingebracht, wobei dann Acrolein freigesetzt wird.

## Beschreibung

Die Erfindung richtet sich auf ein Mittel zum Dotieren wäßriger Systeme mit Acrolein in biozid wirksamer Konzentration, um eine Veralgung, Verkrautung und Verschleimung zu vermeiden.

Acrolein ist ein wohlbekanntes Biozid, um Flüssigkeiten, insbesondere wäßrige Lösungen offener und geschlossener Kreislaufsysteme, welche schleimbildende Mikroorganismen enthalten, zu behandeln. Die biozide Wirksamkeit von Acrolein richtet sich auf die Verhütung, Regulierung und Zerstörung von Mikroorganismen aus der Reihe der Bakterien, Viren, Pilze und Algen; die hohe Effektivität von Acrolein gestattet es, Acrolein in sehr niedriger Anwendungskonzentration in Wasser zu verwenden - US 2,959,476 und US 3,250,667.

Wegen seiner Fähigkeit, nicht nur bei Algen, sondern auch bei höherzelligen Wasserunkräutern die Photosynthese in kurzer Zeit zu stoppen und so ein Absterben zu bewirken, wird Acrolein auch zur Unkrautbekämpfung in Wasserkanälen eingesetzt, um ein Verstopfen der Pumpsysteme zu verhindern. Darüber hinaus hat es sich seit vielen Jahren bei der Ölförderung als sehr effektiver Schwefelwasserstofffänger bewährt, da ei sowohl H₂S irreversibel binden kann als auch die Entstehungsursache durch Abtötung sulfatreduzierender Bakterien (beispielsweise Desulvovibrio desulfuricans) zu beseitigen vermag.

Trotz der hohen bioziden Wirksamkeit des Acroleins stehen einer breiten Anwendung seine Gefahreneigenschaften gegenüber: Acrolein ist giftig und inhalationstoxisch, stechend und tränenreizend sowie leicht entzündlich (Flammpunkt -29 °C, Siedepunkt 53 °C), was die Handhabung problematisch, zumindest aber aufwendig werden läßt; durch Kontamination des Acroleins mit Verunreinigungen besteht ferner explosionsartige Polymerisationsgefahr. Es hat daher nicht an Vorschlägen gefehlt, das Gefahrenpotential beim Umgang mit Acrolein zu reduzieren.

Aus der US 4,851,583 ist bekannt, anstelle Acrolein ein Acroleinacetal einzusetzen und dieses katalytisch zu Acrolein zu hydrolysieren. Zur Dotierung strömender Gewässer ist dieses Verfahren aber nicht geeignet, weil es rasch zur Verschmutzung beziehungsweise Inaktivierung des eingesetzten Ionenaustauschers käme.

Die DE-OS 40 38 471 beschreibt ein Verfahren, bei dem Acrolein aus cyclischen Acetalen durch Zusatz einer Protonensäure unter gleichzeitiger Einwirkung von Wärme und vermindertem Druck oder Durchleiten von Stickstoff freigesetzt, in gasförmigem Zustand abgetrieben und so in das zu dotierende Medium eingeleitet wird. Eine Ausführungsform zur Dotierung von Bewässerungskanälen unter Verwendung eines Acroleinacetals, die nicht auf die Verfügbarkeit elektrischer Energie angewiesen ist, lehrt die DE-Patentanmeldung P 43 26 575.8.

Alle von einem Acroleinacetal ausgehenden Verfahren zum Dotieren wäßriger Systeme haben zudem den Nachteil, daß das Acetal, das selbst aus Acrolein hergestellt wird, wesentlich teurer ist als Acrolein.

Die Aufgabe der vorliegenden Verbindung besteht somit darin, ein Mittel aufzuzeigen, das zum Dotieren wäßriger Systeme geeignet ist, das weniger problematisch zu handhaben ist als flüssiges Acrolein und zudem nicht die mit der Verwendung von Acroleinacetalen verbundenen Nachteile aufweist.

Gefunden wurde ein Mittel zum Dotieren wäßriger Systeme mit Acrolein in biozid wirksamer Konzentration, gekennzeichnet durch einen Gehalt an Acrolein in einer Menge von 30 bis 95 Gew.-% und einen Gehalt an einem oder mehreren anorganischen Trägermaterialien, welche gegenüber Acrolein im wesentlichen inert sind und in 5 %iger Dispersion in Wasser einen pH-Wert von 3 bis 7 aufweisen, in einer Menge von 5 bis 70 Gew.-%.

Das Mittel weist vorzugsweise eine feste, etwa pulverförmige Konsistenz auf; selbstverständlich kann das Mittel auch in Form von geformten Stücken, etwa granulatförmigen Körnern, Tabletten, Pellets, Eiern, Briketts oder anders geformten Preßlingen vorliegen. Bei sehr hohem Acroleingehalt, etwa 85 bis 95 Gew.-%, kann das Mittel auch feucht-krümelig oder hochviskos, gegebenenfalls auch gallertförmig sein. Bei der erfindungsgemäßen Auswahl des Trägermaterials ist das Stoffgemisch lagerstabil.

Ein Auslaufen aus gegebenenfalls beschädigten Gebinden und Vorrichtungen, welches bei Acrolein als eine niedrigviskose, niedrigsiedende Flüssigkeit zu erheblichen Problemen führen kann, ist bei der genannten Konsistenz des erfindungsgemäßen Mittels weniger kritisch, weil es leicht aufgenommen und der Verwendung zugeführt werden kann. Eine örtliche Kontamination des Mittels mit einer Verunreinigung ist im allgemeinen, insbesondere wenn das Mittel in Form geformter Stücke vorliegt, weniger kritisch als im Falle des flüssigen Acroleins, weil das Trägermaterial phlegmatisierend wirkt und zudem eine Weiterleitung von Störeinflüssen im Falle des in geformten und vorzugsweise, wie noch gezeigt wird, zusätzlich umhüllten Mittels gemindert wird. Acrolein wird beim Einbringen des Mittels in Wasser vollständig freigesetzt und als Biozid verfügbar, ohne daß es spezieller Verfahren und Vorrichtungen bedarf, wie im Falle der Verwendung von Acroleinacetalen.

Das Trägermaterial kann aus einem oder mehreren Stoffen bestehen; bevorzugt wird meist ein einheitlicher Stoff. Der pH-Wert einer 5 %igen Dispersion des Trägermaterials in Wasser darf nicht über 7 liegen, weil Acrolein im alkalischen Bereich polymerisationsfähig ist; vorzugsweise liegt der pH-Wert zwischen 5 und 7, insbesondere zwischen 5 und 6,5; der pH-Wert läßt sich gemäß DIN ISO 787/IX beziehungsweise ASTM D1208 bestimmen. Die nach BET bestimmte Oberfläche (DIN 66131) des Trägermaterials liegt vorzugsweise zwischen 100 und 700 m²/g, insbesondere zwischen 150 und 700 m²/g. Unter dem Hinweis "im wesentlichen inert gegenüber Acrolein" wird verstanden, daß das Trägermaterial keine Polymerisation des Acroleins auslösen darf, andererseits eine partielle Umsetzung des Acroleins mit an der Oberfläche des Trägers haftenden funktionellen Gruppen, wie etwa Hydroxyl- oder Carboxylgruppen, nicht ausgeschlossen wird.

Im Prinzip kommen alle Trägermaterialien infrage, welche ein ausreichendes Adsorptionsvermögen für Acrolein aufweisen und den vorgenannten Kriterien genügen. Bevorzugte Trägermaterialien haben oxidische oder silikatische Struktur; der erforderliche pH-Wert kann auch durch eine Behandlung des Trägers mit einer Säure - etwa TiO₂ mit Phosphorsäure und nachfolgender thermischer Behandlung und Auswaschen - erzielt worden sein. Besonders geeignete Trägermaterialien sind pyrogene und gefällte Kieselsäuren und neutral bis sauer wirkende Silikate, darunter Zeolithe, wie insbesondere dealuminierte γ-Zeolithe.

Eine bevorzugte Ausführungsform des Mittels besteht im wesentlichen aus 40 bis 85 Gew.-% Acrolein, 60 bis 15 Gew.-% einer oder mehrerer Kieselsäuren oder Silikate mit einer BET-Oberfläche von 150 bis 700 m²/g und einem pH-Wert in 5 %iger Dispersion von 5 bis 7 und einer wirksamen Menge eines oder mehrerer üblicher Stabilisatoren für Acrolein, vorzugsweise Hydrochinon in einer Menge zwischen 100 und 5000 ppm. Der Begriff "im wesentlichen" bedeutet, daß zusätzlich übliche Verunreinigungen handelsüblichen Acroleins und des Trägermaterials, wie insbesondere Wasser in einer Menge bis 3 Gew.-%, bezogen auf die Summe aus Acrolein und Trägermaterial, ferner Verarbeitungshilfsmittel, wie etwa Tablettensprengmittel, in einer Menge bis 3 Gew.-.%, bezogen auf die Summe aus Acrolein und Trägermaterial, sowie zusätzlich ein Hüllmaterial zur Umhüllung geformter Stücke zwecks zusätzlichen Schutzes und leichterer Handhabung derselben anwesend sein können.

Gemäß einer bevorzugten Ausführungsform liegt das Mittel in Form geformter Stücke mit fester oder gallertförmiger Konsistenz vor, welche eine im wesentlichen Acroleindampf undurchlässige Umhüllung aufweisen. Die Masse der Stücke kann im Bereich von 0,1 g bis mehrere kg liegen, vorzugsweise zwischen 0,2 g und 200 g, insbesondere zwischen 1 g und 50 g. Die Umhüllung erleichtert die problemlose Handhabung und vermindert die Gefahr einer Kontamination.

Zum Gebrauch des Mittels kann die Umhüllung, beispielsweise eine zugeschweißte Folie, entweder entfernt oder durch Perforation für Wasser penetrationsfahig gemacht werden.

Gemäß einer besonders vorteilhaften Ausführungsform sind die bereits erwähnten geformten Stücke mit einem solchen Hüllmaterial umhüllt, das zwar für Acroleindampf weitgehend undurchlässig, aber in Wasser löslich ist. Damit ist eine problemlose Handhabung gewährleistet, weil das im Mittel enthaltene Acrolein erst nach Einbringen des umhüllten Mittels in das zu dotierende Wasser nach ausreichender Auflösung der Umhüllung freigesetzt wird. Ein besonders geeignetes Hüllmaterial ist Gelatine, insbesondere Weichgelatine. Weitere geeignete wasserlösliche oder penetrationsfähige Hüllmaterialien sind Polyalkylenglykole.

Die Umhüllung erfolgt zweckmäßigerweise mittels einer dünnen Folie des Hüllmaterials, welches rundum verschweißt wird, oder durch einen Beschichtungsvorgang. Im Falle eines pulverförmigen, als Preßling geformten oder hochviskosen Gemisches aus Acrolein, Trägermaterial und gegebenenfalls Hilfsstoffen läßt sich dieses unter Verwendung üblicher Verkapselungstechniken umhüllen beziehungsweise verkapseln.

Sofern eine wasserunlösliche Umhüllung zur Anwendung kommt, ist es zweckmäßig, diese so auszugestalten, daß sie unmittelbar vor der Verwendung des umhüllten Mittels zur Dotierung wäßriger Systeme für Wasser penetrationsfähig wird, etwa durch Perforieren oder Öffnen dafür vorgesehener Vorrichtungen in der Umhüllung.

Mittel mit hochviskoser Konsistenz können auch eine Umhüllung in Form eines Druckbehälters oder in Form von Kartuschen aufweisen, aus welchen sie unmittelbar in das zu dotierende wäßrige System eingebracht werden.

Zur Herstellung der erfindungsgemäßen Mittel bieten sich insbesondere zwei alternative Verfahren an:
(i) Herstellung eines homogenen Gemischs aus Acrolein, Trägermaterial und, soweit erwünscht und erforderlich, Hilfsstoffen in einer Intensivmischvorrichtung; soweit erwünscht, schließt sich eine Überführung des Gemischs in geformte Stücke, etwa Preßlinge, und/oder eine Umhüllung mittels üblicher Beschichtungs-, Verkapselungs- oder anderer Umhüllungstechniken oder Einbringen in Druckbehälter oder Kartuschen an. Sofern das Stoffgemisch nach der Homogenisierung verpreßt wird, empfiehlt es sich, die Acroleinkonzentration in Abhängigkeit vom gemischten Preßdruck der Formgebungsvorrichtung so zu begrenzen, üblicherweise auf 30 bis 40 Gew.-% bei Verwendung von Tablettenpressen, daß kein Acrolein abgepreßt wird.
(ii) Aus pulverförmigem Trägermaterial werden in an sich bekannter Weise, gegebenenfalls unter Mitverwendung eines Gleit-, Verbackungs- und Tablettensprengmittels, Preßlinge erzeugt; anschließend wird auf die Preßlinge Acrolein aufgebracht, vorzugsweise in einer solchen Menge,
daß sich die Preßlinge danach noch trocken anfühlen. Auch hier kann sich als weiterer Schritt eine Umhüllung anschließen, etwa durch Tauchen der Acrolein enthaltenden Preßlinge in ein das Hüllmaterial enthaltendes Bad oder durch Aufsprühen des Hüllmaterials in flüssiger Form oder durch Umschweißen mittels einer Folie.

Die erfindungsgemäßen Mittel lassen sich zur Dotierung wäßriger Systeme, insbesondere Wasserkreisläufen, Bewässerungskanälen und Ölfeldwässern, verwenden. Die leicht handhabbaren Mittel werden einfach in das Wasser eingebracht, wobei Acrolein freigesetzt wird. Das Trägermaterial ist inert und führt in dem behandelten wäßrigen System zu keinen Problemen. Wie bereits ausgeführt, ist im Falle der Verwendung von Mitteln mit einer wasserunlöslichen Umhüllung diese vor Gebrauch für Wasser penetrationsfähig zu machen oder zu entfernen.

Es war nicht vorhersehbar, durch Adsorption von Acrolein an einem Trägermaterial zu Mitteln zu gelangen, welche lagerstabil und in vielfacher Hinsicht leichter und sicherer zu handhaben sind als Acrolein. Diese Vorteile werden bei den eine Umhüllung, insbesondere eine solche aus acroleindampfdichtem und wasserlöslichem Material, aufweisenden Mitteln wesentlich gesteigert, da derartige Mittel weitgehend unempfindlich gegen eine unbeabsichtigte Kontamination und zudem im wesentlichen geruchsfrei sind.

### Beispiel 1

50 g einer gefällten Kieselsäure mit den Kenndaten pH 6,5; BET-Oberfläche 450 m²/g, im Handel erhältich unter dem Namen Sipernat® 50 (Degussa), werden mit 117 g Acrolein, das mit 1100 ppm Hydrochinon stabilisiert wurde, gemischt. Man erhält 167 g eines freifließenden Pulvers. Der Acroleingehalt beträgt 70 Gew.-%.

### Beispiel 2

50 g einer gefällten Kieselsäure mit den Kenndaten pH 7,0; BET-Oberfläche 650 m²/g (Handelsprodukt Sipernat FK 310, Degussa) werden mit 93 g stabilisiertem Acrolein vermischt. Man erhält 143 g eines freifließenden Pulvers mit der Acroleingehalt von 65 Gew.-%.

### Beispiel 3

75 g eines durch Hochtemperaturhydrolyse erzeugten Siliciumdioxids mit den Kenndaten pH ca. 4; BET-Oberfläche 200 m²/g (Handelsprodukt Aerosil® 200, Degussa) werden mit 425 g stabilisiertem Acrolein gemischt. Man erhält 500 g eines durchscheinenden, opaken Körpers mit gummiartiger, pumpfähiger Konsistenz und einem Acroleingehalt von 85 Gew.-%.

### Beispiel 4

Die Acrolein-Kieselsäuremischung aus Beispiel 1 wird in handelsüblichen Hartgelatine-Kapseln mit einem Kapsel-Füllgerät (Fa. Roebiger, Berlin) verkapselt. Die erhaltenen praktisch geruchlosen Kapseln erweisen sich als lagerstabil.

Nach Einbringen der Kapseln in Wasser lösen sich diese innerhalb etwa einer Stunde auf, so daß Acrolein freigesetzt wird.

### Beispiel 5

5,0 g einer gefällten Kieselsäure mit einer Handpresse bei 5 t Druck zu einer Tablette verpreßt. Durch Auftropfen von 5,5 g Acrolein auf diese Tablette wird ein Mittel mit einem Acroleingehalt von 52 Gew.-% erhalten. Durch Tauchen der gekühlten Tablette in flüssiges Paraffinwachs kann ein wasserdichter Überzug aufgebracht werden.

### Beispiel 6

Zu 30 g eines dealuminierten γ-Zeoliths mit der Nennweite 7,4 Å, pH 4,5, Modul 200, das in Zylinderform vorliegt, werden 19,5 g stabilisiertes Acrolein getropft. Man erhält ein optisch trockenes Granulat mit 30 Gew.-% Acrolein.

## Patentansprüche

1. Mittel zum Dotieren wäßriger Systeme mit Acrolein in biozid wirksamer Konzentration, gekennzeichnet durch einen Gehalt an Acrolein in einer Menge von 30 bis 95 Gew.-%. und einen Gehalt an einem oder mehreren anorganischen Trägermaterialien, welche gegenüber Acrolein im wesentlichen inert sind und in 5 %iger Dispersion in Wasser einen pH-Wert von 3 bis 7 aufweisen, in einer Menge von 5 bis 70 Gew.-%.

2. Mittel nach Anspruch 1,
dadurch gekennzeichnet,
daß es als Trägermaterial eine Kieselsäure und/oder ein Silikat, welche eine Oberfläche nach BET von 100 bis 700 m²/g aufweisen, enthält.

3. Mittel nach Anspruch 1 oder 2,
dadurch gekennzeichnet,
daß es im wesentlichen aus 40 bis 85 Gew.-% Acrolein, 60 bis 15 Gew.-% einer oder mehrerer Kieselsäuren und/oder Silikate, welche eine BET-Oberfläche von 150 bis 700 m²/g und einen pH-Wert in 5 %iger Dispersion von 5 bis 7 aufweisen, und einer wirksamen Menge eines oder mehrerer Stabilisatoren für Acrolein besteht.

4. Mittel nach einem der Ansprüche 1 bis 3,
dadurch gekennzeichnet,
daß es eine feste Konsistenz aufweist und in Form geformter Stücke vorliegt.

5. Mittel nach einem der Ansprüche 1 bis 4,
dadurch gekennzeichnet,
daß es in Form geformter fester oder gallertförmiger Stücke vorliegt, welche von einer wasserlöslichen oder wasserpenetrationsfähigen, im wesentlichen Acroleindampf undurchlässigen Umhüllung umgeben sind.

6. Mittel nach Anspruch 5,
dadurch gekennzeichnet,
daß es sich bei den umhüllten geformten Stücken um umhüllte Preßlinge oder um mit pulver- oder gallertförmigem Stoffgemisch gefüllte Kapseln handelt, wobei das Kapsel- oder Hüllmaterial aus Gelatine, insbesondere Weichgelatine oder aus Polyethylenglykol, besteht.

7. Verfahren zur Herstellung des Mittels gemäß Anspruch 1 bis 4,
dadurch gekennzeichnet,
daß man Acrolein und das Trägermaterial sowie, sofern anwesend, Hilfs- und Zusatzstoffe in einer Intensivmischvorrichtung mischt und homogenisiert und bei Bedarf das erhaltene Gemisch verkapselt oder in Formkörper überführt und diese mit einem Hüllmaterial umhüllt.

8. Verfahren zur Herstellung des Mittels gemäß Anspruch 1 bis 4,
dadurch gekennzeichnet,
daß man Acrolein auf Formkörper aus im wesentlichen dem Trägermaterial aufbringt und bei Bedarf die so erhaltenen Mittel mit einem Hüllmaterial umhüllt.

9. Verwendung von Mitteln gemäß einem der Ansprüche 1 bis 5 zum Dotieren wäßriger Systeme mit Acrolein in biozid wirksamer Konzentration.
